# EUROPEAN PATENT APPLICATION

(11) **EP 2 418 490 A1**
(43) Date of publication of application: **15.02.2012**
(21) Application number: 10761709.4
(22) Date of filing: 07.04.2010
(51) Int. Cl.: G01N 33/574, C12Q 1/68, G01N 33/53, C12N 15/09

(54) **TUMOR MARKER AND USE THEREOF**

(30) Priority: 10.04.2009 JP 2009095561
(71) Applicant: LSIP, LLC, Chiyoda-ku Tokyo 100-0005 (JP)
(72) Inventor: ICHIMIYA, Shingo, Sapporo-shi Hokkaido 060-8556 (JP); KIKUCHI, Tomoki, Sapporo-shi Hokkaido 060-8556 (JP); TONOOKA, Akiko, Sapporo-shi Hokkaido 060-8556 (JP); SATOH, Noriyuki, Sapporo-shi Hokkaido 060-8556 (JP)
(74) Representative: Cabinet Plasseraud
(86) International application number: PCT/JP2010/056293
(87) International publication number: WO 2010/117010

(57) **Abstract**

The present invention detects SNX5 in a sample from a subject. The SNX5 is used as a tumor marker specific to papillary thyroid carcinoma, whereby diagnosis of papillary thyroid carcinoma is carried out easily. The present invention provides also a technique of discriminating papillary thyroid carcinoma using the tumor marker.

## Description

### Technical Field

The present invention relates to a tumor marker and its use. More specifically, the present invention relates to a method of detecting a tumor marker and a tool for use in the method, each of which is suitable for diagnosis of papillary thyroid carcinoma.

### Background Art

Papillary thyroid carcinoma is the most common malignant tumor of thyroid carcinomas. Metastases of the papillary thyroid carcinoma to lung or lymph node can lead to a poor prognosis. Therefore, a definitive diagnosis of papillary thyroid carcinoma is very important. In the definitive diagnosis of papillary thyroid carcinoma, a histopathologic evaluation is essential. However, since there exist a variety of malignant tumors that show papillary structures besides papillary thyroid carcinoma, it is very difficult not only to determine whether or not the lesion is primary, but also to determine a neoplastic lesion which has metastasized to lymph node.

The histopathological characteristics of papillary thyroid carcinoma are evaluated in terms of expression of molecules specific to thyroid tissue. For example, thyroglobulin and thyroid transcription factor 1 (TTF-1) are known as thyroid markers. The thyroglobulin is a dimeric glycoprotein secreted from thyroid tissue. It is known that the thyroglobulin level increases in cells or blood in the case of thyroid disease.

### Citation List

### Patent Literatures

Patent Literature 1
PCT International Publication WO2006/133361 (Publication Date: December 14, 2006)
Patent Literature 2
Japanese Translation of PCT Patent Application, Tokuhyo, No. 2008-500033 A (Publication Date: January 10, 2008)

### Non Patent Literatures

Non Patent Literature 1
Otsuki, T et al., SNX5, a New Member of the Sorting Nexin Family, Binds to the Fanconi Anemia Complementation Group A Protein. Biochemical and Biophysical Research Communications 265: 630-635 (1999)
Non Patent Literature 2
Teasdale, R.D. et al., A large family of endosome-localized proteins related to sorting nexin 1. Biochem. J. 358: 7-16 (2001)

### Summary of Invention

### Technical Problem

Measurement of the thyroglobulin level in blood has been employed in postoperative follow-ups (especially for checking for metastasis) of thyroid carcinoma. However, the measurement of the thyroglobulin level does not allow for determination of whether the thyroid carcinoma is benign or malignant. Further, some of the molecules reported to be expressed specifically in thyroid tissue is expressed also in other organ(s). Therefore, an excellent tumor marker specific to papillary thyroid carcinoma is strongly demanded.

The present invention has been made in view of the above problem, and an object of the present invention is to provide a tumor marker specific to papillary thyroid carcinoma and a technique of discriminating papillary thyroid carcinoma by using the tumor marker.

### Solution to Problem

The inventors of the present invention have produced an antibody to a human SNX5 protein, and carried out immunohistochemical staining with respect to various normal tissues and neoplastic lesions of humans with use of the anti-human SNX5 antibody. As a result, the inventors have found that the SNX5 reacts specifically with thyroid follicular epithelium, especially strongly reacts with papillary thyroid carcinoma which is a malignant tumor of thyroid origin. The inventors have also found that little expression of SNX5 is detected in thyroid carcinomas of other tissue types, papillary adenocarcinomas of gastrointestinal tract origin, and papillary adenocarcinomas of lung origin. As a result, the inventors have completed the present invention.

That is, a method in accordance with the present invention is a method of detecting a tumor marker, which method includes the step of detecting SNX5 in a sample from a subject.

The method in accordance with the present invention is preferably configured such that the sample is (i) a tissue collected from a subject or (ii) a culture or a section of the tissue. Note, however, that the sample may be a cell lysate prepared from a tissue collected from a subject or prepared from a culture of the tissue. Further, the tissue is preferably a thyroid tissue. Alternatively, the tissue may be a body fluid such as blood.

The method in accordance with the present invention is preferably configured such that the subject is a patient with suspected thyroid disease. Note, however, that the subject may be a patient who has already developed papillary thyroid carcinoma.

The SNX5 is preferably a SNX5 protein or a fragment thereof. In this case, the step of detecting SNX5 is preferably an immunoassay using an anti-SNX5 antibody. The anti-SNX5 antibody to be used is preferably an anti-human SNX5 antibody, more preferably an anti-human SNX5 monoclonal antibody, and most preferably a mouse anti-human SNX5 monoclonal antibody produced by a hybridoma 48C2 or a monoclonal antibody having a binding activity equivalent to that of the mouse anti-human SNX5 monoclonal antibody.

Alternatively, the SNX5 may be a SNX5 gene or a fragment thereof. In this case, the step of detecting SNX5 is preferably a step of hybridizing a nucleic acid probe with the SNX5 gene or the fragment thereof. The nucleic acid probe is preferably a hybridization probe. Alternatively, the nucleic acid probe may be a PCR primer.

It is more preferable that the method in accordance with the present invention include both the step of detecting a SNX5 protein or a fragment thereof and the step of detecting a SNX5 gene or a fragment thereof.

The above method can serve as a method of providing a diagnostic criterion for papillary thyroid carcinoma, and can serve also as a method of providing a criterion for distinguishing a malignant tumor in a papillary form. Further, the method can serve as a method of providing a criterion for determining whether or not a lesion of papillary thyroid carcinoma is primary. Furthermore, the method can serve as a method of providing a criterion for determining whether or not a tumor metastasized to cervical lymph node is of thyroid origin.

A kit in accordance with the present invention is a kit for detecting a tumor marker, which kit includes an anti-SNX5 antibody. The kit in accordance with the present invention preferably further includes a reagent for detecting the anti-SNX5 antibody. The anti-SNX5 antibody is preferably an anti-human SNX5 antibody, more preferably anti-human SNX5 monoclonal antibody, and most preferably a mouse anti-human SNX5 monoclonal antibody produced by a hybridoma 48C2 or a monoclonal antibody having a binding activity equivalent to that of the mouse anti-human SNX5 monoclonal antibody.

A kit in accordance with the present invention is a kit for detecting a tumor marker, which kit includes an oligonucleotide that can hybridize with a SNX5 gene or a fragment thereof. The kit in accordance with the present invention preferably further includes a reagent for detecting the oligonucleotide. The oligonucleotide is preferably a hybridization probe for the SNX5 gene or the fragment thereof. Alternatively, the oligonucleotide may be a PCR primer.

The kit preferably serves as a kit for providing a diagnostic criterion for papillary thyroid carcinoma. Note, however, that the kit can serve also as a kit for providing a criterion for distinguishing a malignant tumor in a papillary form. Further, the kit can serve as a kit for providing a criterion for determining whether or not a lesion of papillary thyroid carcinoma is primary. Furthermore, the kit can serve as a kit for providing a criterion for determining whether or not a tumor metastasized to cervical lymph node is of thyroid origin.

For a fuller understanding of the other objects, nature and advantages of the invention, reference should be made to the ensuing detailed description taken in conjunction with the accompanying drawings.

### Advantageous Effects of Invention

By use of the present invention, it is possible to detect a tumor marker that is suitable for diagnosis of papillary thyroid carcinoma.

### Brief Description of Drawings

Fig. 1

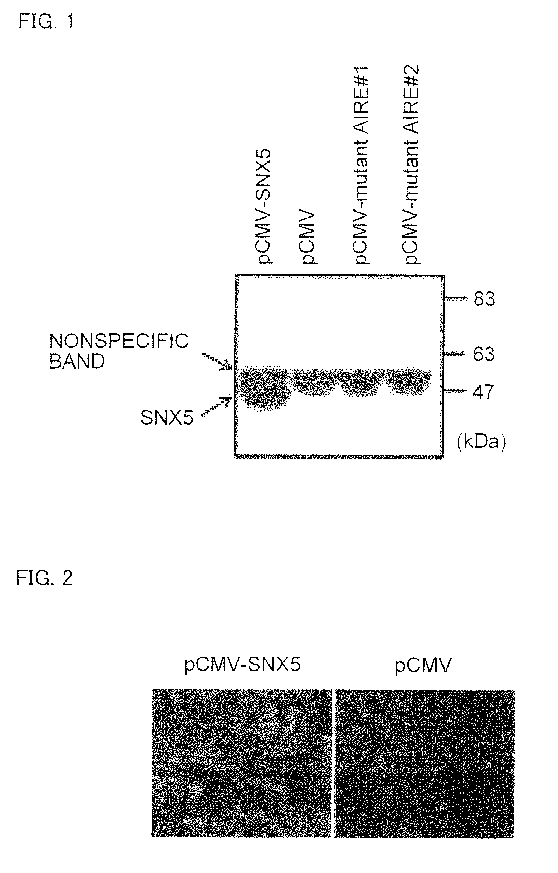

   Fig. 1 is a view showing the result of western blotting, using an anti-human SNX5 monoclonal antibody, which was carried out with respect to a soluble fraction of a human 293 cell into which pCMV-SNX5 has been introduced. Controls used here were human 293 cells into which pCMV, pCMV-mutant AIRE#1 and pCMV-mutant AIRE#2 have been introduced, respectively.
Fig. 2
   Fig. 2 is a view showing the result of immunohistochemical staining, using an anti-human SNX5 monoclonal antibody, which was carried out with respect to human 293 cells into which pCMV-SNX5 has been introduced. Controls used here were human 293 cells into which pCMV has been introduced. Each cell was fixed with paraformaldehyde.
Fig. 3

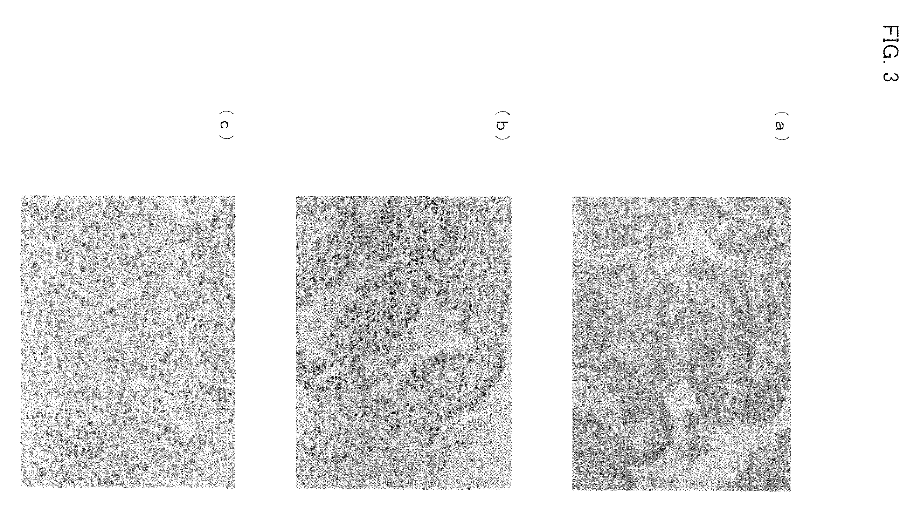

   Fig. 3 is a view showing the result of immunohistochemical staining, using an anti-human SNX5 monoclonal antibody, which was carried out with respect to formalin-fixed, paraffin-embedded sections of human adenocarcinoma tissue. (a) of Fig. 3 shows the result of immunohistochemical staining of papillary thyroid carcinoma. (b) of Fig. 3 shows the result of immunohistochemical staining of lung adenocarcinoma. (c) of Fig. 3 shows the result of immunohistochemical staining of mammary adenocarcinoma.
Fig. 4

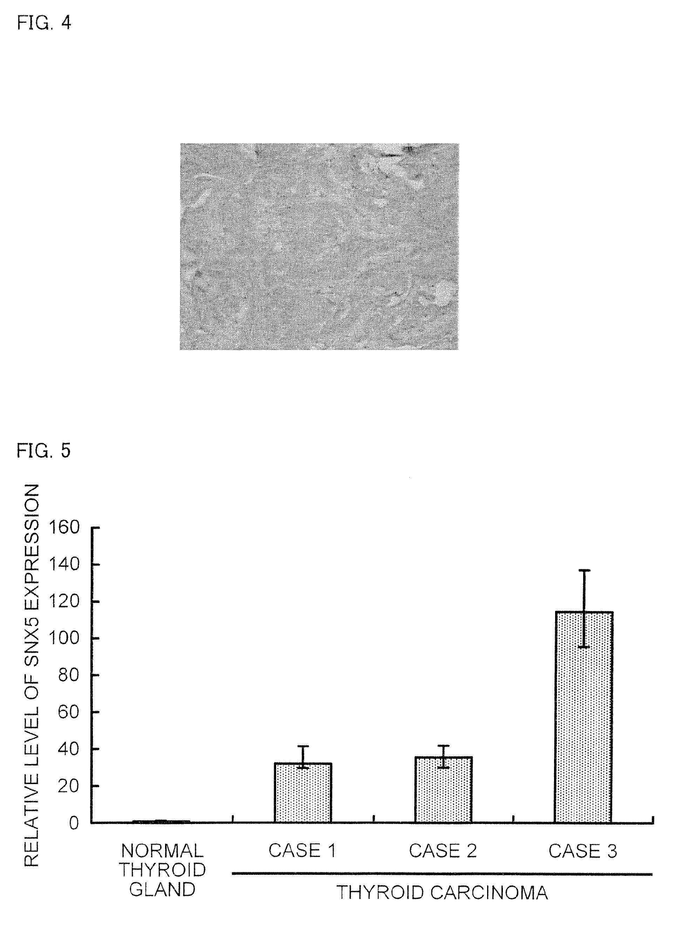

   Fig. 4 is a view showing the result of immunohistochemical staining, using an anti-human SNX5 polyclonal antibody, which was carried out with respect to a formalin-fixed, paraffin-embedded section of human adenocarcinoma tissue.
Fig. 5
   Fig. 5 is a view showing the result of quantitative RT-PCR, which was carried out with respect to human SNX5 genes in normal tissue of thyroid gland and cancer tissue of thyroid gland.

### Description of Embodiments

As described earlier, the inventors of the present invention have found that (i) high expression of SNX5 is detected in papillary thyroid carcinoma, which is a malignant tumor of thyroid origin but (ii) little expression of SNX5 is detected in thyroid carcinomas of other tissue types, papillary adenocarcinomas of gastrointestinal tract origin, and papillary adenocarcinomas of lung origin.

A human sorting nexin-5 (SNX5) is a molecule involved in endoplasmic reticulum transport within a cell, and has been suggested as being closely related to cell migration (for example, refer to Non Patent Literatures 1 and 2). Further, Patent Literature 1 lists many genes possibly related to multiple myeloma, one of which genes is SNX5. Patent Literature 2 lists many possible tumor-associated peptides (antigenic peptides) that bind to MHC molecules, one of which tumor-associated peptides is a partial fragment (amino acids 292 to 300: SEQ ID NO:524 of Patent Literature 2) of a SNX5 protein. Note, however, that the specificity of SNX5 to papillary thyroid carcinoma is neither disclosed nor suggested in any of the literatures. As is clear from this, the present invention provides an excellent tumor marker for papillary thyroid carcinoma.

### [1. Tumor marker]

The present invention provides an excellent tumor marker for papillary thyroid carcinoma. In one embodiment, the tumor marker of the present invention is a SNX5 protein or a fragment thereof. In another embodiment, the tumor marker of the present invention is a SNX5 gene or a fragment thereof.

The SNX5 protein as used in this Description is (i) a polypeptide having an amino acid sequence shown in SEQ ID NO:1 or (ii) a polypeptide which has the same amino acid sequence as shown in SEQ ID NO:1 except that one to several amino acids are deleted, substituted and/or added, and which has a SNX5 activity. Alternatively, the SNX5 protein may be (iii) a polypeptide encoded by a polynucleotide having a nucleotide sequence shown in SEQ ID NO:2, (iv) a polypeptide which is encoded by a polynucleotide having the same nucleotide sequence as shown in SEQ ID NO:2 except that one to several nucleotides are deleted, substituted and/or added, and which has a SNX5 activity, (v) a polypeptide which is encoded by a polynucleotide that can hybridize with the polynucleotide having the nucleotide sequence shown in SEQ ID NO:2 under stringent conditions, and which has a SNX5 activity, or (vi) a polypeptide which is encoded by a polynucleotide having a homology of not less than 80% to the polynucleotide having the nucleotide sequence shown in SEQ ID NO:2, and which has a SNX5 activity.

When used in terms of a polypeptide (amino acids), the term "one to several" means preferably 1 to 30, more preferably 1 to 20, further preferably 1 to 10, and still further preferably 1 to 5. Note that a person skilled in the art can easily understand about how many amino acids are intended by the term "one to several", depending on the length of the target polypeptide.

When used in terms of a polynucleotide (nucleotides), the term "one to several" means preferably 1 to 100, more preferably 1 to 50, further preferably 1 to 30, and still further preferably 1 to 15. Note that a person skilled in the art can easily understand about how many nucleotides are intended by the term "one to several", depending on the length of the target polynucleotide. For example, in a case of an oligonucleotide (described later), the term "one to several" means preferably 1 to 10, more preferably 1 to 7, further preferably 1 to 5, and still further preferably 1 to 3.

The homology to the target polypeptide or polynucleotide as used in this Description is preferably not less than 80%, more preferably not less than 85%, further preferably not less than 90%, and still further preferably not less than 95%.

The term "SNX5 activity" as used in this Description means a binding capacity to an anti-SNX antibody, which is produced by using as an antigen the polypeptide having the amino acid sequence shown in SEQ ID NO: 1. The SNX antibody may be of any kind provided that it is an anti-SNX5 antibody (described later), and is preferably a 48C2 antibody (described later). Further, the foregoing polypeptide may be a recombinant human SNX5 protein or an isolated, purified native human SNX5 protein.

The SNX5 gene as used in this Description is (i) a polynucleotide coding for a polypeptide having an amino acid sequence shown in SEQ ID NO:1 or (ii) a polynucleotide coding for a polypeptide which has the same amino acid sequence as shown in SEQ ID NO:1 except that one to several amino acids are deleted, substituted and/or added, and which has a SNX5 activity. Alternatively, the SNX5 gene may be (iii) a polynucleotide having a nucleotide sequence shown in SEQ ID NO:2, (iv) a polynucleotide which has the same nucleotide sequence as shown in SEQ ID NO:2 except that one to several nucleotides are deleted, substituted and/or added, and which codes for a polypeptide having a SNX5 activity, (v) a polynucleotide which can hybridize with the polynucleotide having the nucleotide sequence shown in SEQ ID NO:2 under stringent conditions, and which codes for a polypeptide having a SNX5 activity, or (vi) a polynucleotide which has a homology of not less than 80% to the polynucleotide having the nucleotide sequence shown in SEQ ID NO:2, and which codes for a polypeptide having a SNX5 activity.

SEQ ID NO:1 is an amino acid sequence of SNX5, which amino acid sequence is registered with GenBank (accession No. AF121855). SEQ ID NO:2 is a nucleotide sequence which codes for the amino acid sequence shown in SEQ ID NO:1, and corresponds to an open reading frame (nucleotides at positions 181 to 1395) of a nucleotide sequence (SEQ ID NO:3) of SNX5 which nucleotide sequence is registered with GenBank (accession No. AF121855).

The term "polypeptide" as used in this Description is used interchangeably with a "peptide" or "protein", and means a polymer of amino acids. Further, the "fragment" of the polypeptide means a partial fragment of the polypeptide. The polypeptide in accordance with the present invention may be produced recombinantly, synthesized chemically, or isolated from a natural source.

The term "polynucleotide" as used in this Description is used interchangeably with a "gene", "nucleic acid" or "nucleic acid molecule", and means a polymer of nucleotides. Further, the "fragment" of the polynucleotide means a partial fragment of the polynucleotide. The term "nucleotide sequence" as used in this Description is used interchangeably with a "nucleic acid sequence" or "base sequence".

The polynucleotide in accordance with the present invention can be present in the form of RNA (e.g., mRNA) or in the form of DNA (e.g., cDNA or genome DNA). The DNA can be in either single-stranded or double-stranded form. The single-strand DNA and the RNA each can be a coding strand (also known as sense strand) or can be a noncoding strand (also known as antisense strand).

The term "oligonucleotide" as used in this Description means several to several tens of nucleotides bound to one another, and is used interchangeably with the "polynucleotide".

The term "stringent (hybridization) conditions" as used in this Description means incubating a filter overnight at 42°C in a hybridization solution (containing 50% formamide, 5× SSC (150 mM of NaCl and 15 mM of trisodium citrate), 50 mM of sodium phosphate (pH 7.6)), 5× Denhart's liquid, 10% dextran sulfate, and 20 µg/ml of denatured, sheared salmon sperm DNA) and thereafter washing the filter at about 65°C in a 0.1 × SSC. Note, however, that the washing condition at high stringency is changed as needed depending on the polynucleotide to be hybridized. For example, in a case of using a DNA derived from a mammal, it is preferable to wash the DNA at 65°C in a 0.5 × SSC containing 0.1% SDS (preferably 15 minutes × twice). In a case of using a DNA derived from *Escherichia coli,* it is preferable to wash the DNA at 68°C in a 0.1 × SSC containing 0.1% SDS (preferably 15 minutes × twice). In a case of using an RNA, it is preferable to wash the RNA at 68°C in a 0.1 × SSC containing 0.1% SDS (preferably 15 minutes × twice). In a case of using an oligonucleotide, it is preferable to wash the oligonucleotide at a hybridization temperature in a 0.1 × SSC containing 0.1% SDS (preferably 15 minutes × twice).

By use of the tumor maker of the present invention, it is possible to provide a criterion for a malignant tumor in a papillary form. This allows for easy determination of whether or not the malignant tumor is papillary thyroid carcinoma. Further, the present invention is very useful for determining whether or not a lesion is primary. Moreover, by use of the present invention, it is possible to more clearly determine whether a tumor metastasized to cervical lymph node is of thyroid origin or originates in other tissue (e.g., lung, mammary gland).

### [2. Method of detecting tumor marker]

The present invention provides a method of detecting a tumor marker. The method of detecting the tumor marker in accordance with the present invention includes the step of detecting SNX5 in a subject sample (a sample from a subject). In one embodiment, the method in accordance with the present invention includes the step of detecting a SNX5 protein or a fragment thereof. In another embodiment, the method in accordance with the present invention includes the step of detecting a SNX5 gene or a fragment thereof.

The "subject sample" as used in this Description means any tissue (including body fluids such as blood) or cell collected from a subject. The "subject sample" can encompass also a section or a cell lysate, which is prepared from the above tissue or cell. The subject sample preferred in the present invention is for example, but not limited to, tumor tissue or serum. It should be noted that the step of taking out a tissue or cell directly from a subject, which step is a first stage of the sample collection, is to be carried out by a doctor and is not encompassed in the present invention. Further, the step of determining whether or not the subject is affected by the disease on the basis of the result obtained through the method of the present invention is also to be carried out by a doctor, and is not encompassed in the present invention.

The subject to be dealt with in the present invention is preferably a patient with suspected thyroid disease, and more preferably a patient who has developed papillary thyroid carcinoma. Note, however, that the subject may be a healthy subject. In the method in accordance with the present invention, the preferred subject sample is, but not limited to, a thyroid tissue collected from a patient with suspected thyroid disease or a culture of the thyroid tissue, or a tissue section or cell lysate prepared from the thyroid tissue or the culture thereof. A person skilled in the art easily understands that how to collect a sample can be determined as needed depending on the target tissue or cell.

In an embodiment in which a SNX5 protein is to be detected, the step of detecting SNX5 can be an immunoassay using an anti-SNX5 antibody. The term "immunoassay" as used in this Description means an assay carried out by utilizing an immunological binding reaction based on an antigen-antibody reaction. Examples of the assay carried out by utilizing the immunological binding reaction include immunohistochemistry, immunoelectron microscopy, western blotting, immunoprecipitation, sandwich ELISA assay, radioimmunoassay, and antibody assay (e.g., immunodiffusion assay), and affinity chromatography. These techniques are well known in the field of the present invention, and therefore a person skilled in the art can easily implement the present invention.

The anti-SNX5 antibody is preferably an anti-human SNX5 antibody, more preferably a monoclonal antibody, and further preferably a mouse anti-human SNX5 monoclonal antibody (also referred to as 48C2 antibody) produced by a hybridoma 48C2. The inventors of the present invention have found that the 48C2 antibody recognizes the N-terminal side (positions 1 to 177 of SEQ ID NO: 1, i.e., SEQ ID NO:10) of the human SNX5 protein (the results are omitted here). Specifically, the "fragment of the SNX5 protein" is preferably a polypeptide which has part of the amino acid sequence shown in SEQ ID NO: 1, and which has an amino acid sequence shown in SEQ ID NO:10 or has an amino acid sequence having at least 8 consecutive amino acids of the amino sequence shown in SEQ ID NO: 10. Note that a person skilled in the art who has read this Description easily understands that a monoclonal antibody having a binding activity equivalent to that of the 48C2 antibody is also encompassed in the antibody suitable for use in the present invention.

In a case where the subject sample is (i) a tissue collected from a subject or (ii) a culture or a section thereof, the immunoassay can be immunohistochemical staining. In a case where the subject sample is a cell lysate prepared from a tissue collected from a subject or a culture thereof, the immunoassay may be western blotting. In a case where the subject sample is blood collected from a subject, the immunoassay can be ELISA. These immunoassays are preferably used for preoperative differential diagnosis, but may be used postoperatively to check for metastasis.

The mouse anti-human SNX5 monoclonal antibody produced by a hybridoma 48C2 is not only suitable for the detection of SNX5 protein by western blotting and the detection of SNX5 protein in a formalin-fixed, paraffin-embedded section, but also allows for identification of SNX5 protein in a paraform-fixed tissue. Regarding a function of SNX5 in cytoplasm which function has been reported hitherto, the present invention shows that SNX5 is localized in cytoplasm even when the mouse anti-human SNX5 monoclonal antibody is used. This supports specificity of the mouse anti-human SNX5 monoclonal antibody. Note that the hybridoma 48C2 is kept under the management of Sapporo Medical University Intellectual Property Management Office (Nishi 17 cho-me, Minami 1 jo, Chuo-ku, Sapporo-shi, 060-8556), and can be shared if needed.

In histopathology of a malignant tumor, immunohistochemical investigation is very important. The use of a tumor marker specific to a particular organ or particular tissue allows for easy definitive diagnosis of a metastatic lesion whose primary site has not yet been determined, and allows for prompt determination of diagnosis or treatment policy. Although there have been found various molecular markers specific to particular organs, only limited kinds of antibodies are practically used in histopathology. In particular, there has not been found an antibody suitable for distinguishing papillary thyroid carcinoma.

Immunohistochemical staining using a mouse anti-human SNX5 monoclonal antibody was carried out with respect to a formalin-fixed, paraffin-embedded section which is for use in usual histopathologic examinations. As a result, although strong expression of SNX5 was detected in a papillary carcinoma of thyroid origin, no expression was detected in a cancer tissue derived from lung carcinoma or breast carcinoma which are typical examples of other adenocarcinoma. For the purpose of verifying these results, immunohistochemical staining using a commercially available rabbit anti-human SNX5 polyclonal antibody was carried out. As a result, high expression of SNX5 was also detected in a tumor cell. As is clear from this, SNX5 is excellent as a tumor marker for papillary thyroid carcinoma. Further, the mouse anti-human SNX5 monoclonal antibody is an excellent detection tool which allows for an immunohistologic analysis of a paraform-fixed tissue.

In an embodiment in which a SNX5 gene is to be detected, the step of detecting SNX5 can be the step of hybridizing a nucleic acid probe with a SNX5 gene or a fragment thereof. The "nucleic acid probe" as used in this Description is not particularly limited provided that the nucleic acid probe can hybridize with a target nucleic acid. The "nucleic acid probe" may be a so-called hybridization probe or a PCR primer. The techniques of hybridization and PCR are well known in the field of the present invention, and therefore a person skilled in the art can easily implement the present invention. In a case where the subject sample is a tissue sample collected from a subject, various methods known in this field can be employed. For example, in situ hybridization or in situ PCR can be employed. In a case where the subject sample is blood collected from a subject, for example usual PCR can be employed.

The nucleic acid probe is not particularly limited provided that it is an oligonucleotide having a partial sequence of the SNX5 gene (or strand complementary thereto). The nucleic acid probe may be (i) an oligonucleotide which has a nucleotide sequence shown in SEQ ID NO:4 or SEQ ID NO:5 or which has a sequence complementary to this nucleotide sequence or (ii) an oligonucleotide which has a nucleotide sequence shown in any one of SEQ ID NOs:6-9 or which has a sequence complementary to this nucleotide sequence. Alternatively, the nucleic acid probe may be an oligonucleotide coding for the "fragment of the SNX5 protein", because the 48C2 antibody recognizes the N-terminal side of the human SNX5 protein.

In a case of in situ hybridization, the nucleic acid probe is preferably an oligonucleotide having 15 to 50 consecutive nucleotides, more preferably 20 to 50 consecutive nucleotides, further preferably 20 to 45 consecutive nucleotides, and most preferably 25 to 40 consecutive nucleotides of the nucleotide sequence shown in SEQ ID NO:1 or a sequence complementary thereto. In a case of PCR (including in situ PCR), the nucleic acid probe is preferably an oligonucleotide having 15 to 50 consecutive nucleotides, more preferably 15 to 40 consecutive nucleotides, further preferably 15 to 35 consecutive nucleotides, and most preferably 25 to 35 consecutive nucleotides of the nucleotide sequence shown in SEQ ID NO:1 or a sequence complementary thereto.

The detection method in accordance with the present invention is characterized by including the step of detecting a SNX5 gene. Note, however, that it is more preferable that the detection method in accordance with the present invention further include the foregoing step of detecting a SNX5 protein.

As has been described, by use of the method of detecting a tumor marker in accordance with the present invention, it is possible to provide a criterion for a malignant tumor in a papillary form. This allows for easy determination of whether or not the malignant tumor is papillary thyroid carcinoma. Further, the present invention is very useful for determining whether or not a lesion is primary. Moreover, by use of the present invention, it is possible to more clearly determine whether a tumor metastasized to cervical lymph node is of thyroid origin or originates in other tissue (e.g., lung, mammary gland). That is, the method in accordance with the present invention can serve as a method of providing a diagnostic criterion for papillary thyroid carcinoma (e.g., method of obtaining data for diagnosis of papillary thyroid carcinoma), and can serve also as a method of providing a criterion for distinguishing a malignant tumor in a papillary form (e.g., method of obtaining data for distinguishing a malignant tumor in a papillary form). Further, the method in accordance with the present invention can serve as a method of providing a criterion for determining whether or not a lesion of papillary thyroid carcinoma is primary (e.g., a method of obtaining data for determining whether or not a lesion of papillary thyroid carcinoma is primary). Furthermore, the method in accordance with the present invention can serve as a method of providing a criterion for determining whether or not a tumor metastasized to cervical lymph node is of thyroid origin (e.g., method of obtaining data for determining whether or not a tumor metastasized to cervical lymph node is of thyroid origin).

### [3. Detection tool for tumor marker]

The present invention provides a kit for detecting a tumor marker. The kit in accordance with the present invention is characterized by including a tool for detecting SNX5 in a subject sample.

The term "kit" as used in this Description means a package including a container (e.g., bottle, plate, tube, or dish) for containing therein a particular material. Note, however, that the term "kit" includes also a single substance serving as a composition, in which a material is contained. The kit preferably includes instructions for use of each material. The term "include (including)" as used in this Description in terms of a kit means a state where something is contained in any of containers constituting the kit. Further, the kit in accordance with the present invention can be a single package constituted by a plurality of different compositions. In a case where the composition(s) is/are in the form of solution, the composition(s) may be contained in the container(s). In the kit in accordance with the present invention, a single container may contain therein a mixture of substances A and B. Alternatively, respective different containers may contain the substances A and B. The "instructions" may be written or printed on a piece(s) of paper or other media, or may be recorded on an electronic medium such as a magnetic tape, computer-readable disc or tape, or a CD-ROM. The kit in accordance with the present invention can include a container containing therein a diluent, solvent, wash liquid or other regent. Further, the kit in accordance with the present invention may include an instrument and regent necessary for collecting a subject sample. Moreover, the kit in accordance with the present invention may include an implement and regent necessary for preparing a section or cell lysate from a subject sample.

In one embodiment, the kit in accordance with the present invention includes an antibody for detecting a SNX5 protein or a fragment thereof. The antibody is preferably an anti-human SNX5 antibody, more preferably an anti-human SNX5 monoclonal antibody, and most preferably a mouse anti-human SNX5 monoclonal antibody produced by a hybridoma 48C2 or a monoclonal antibody having a binding activity equivalent to that of the mouse anti-human SNX5 monoclonal antibody. Note that the kit in accordance with the present invention preferably further includes a regent for detecting the foregoing antibody. In this regard, as described earlier, the kit may be provided in the form of composition that contains an antibody for detecting a SNX5 protein or a fragment thereof.

In another embodiment, the kit in accordance with the present invention includes an oligonucleotide for detecting a SNX5 gene or a fragment thereof. The oligonucleotide is preferably an oligonucleotide that can hybridize with a SNX5 gene or a fragment thereof, and preferably a hybridization probe or a PCR primer for the SNX5 gene or the fragment thereof. Note that the kit in accordance with the present invention preferably further includes a regent for detecting the foregoing oligonucleotide. In this regard, as described earlier, the kit may be provided in the form of composition that contains an oligonucleotide for detecting a SNX5 gene or a fragment thereof.

It is preferable that the kit in accordance with the present invention be a kit for providing a diagnostic criterion for papillary thyroid carcinoma. Note, however, that the kit can serve also as a kit for providing a criterion for distinguishing a malignant tumor in a papillary form. Further, the kit in accordance with the present invention can serve as a kit for providing a criterion for determining whether or not a lesion of papillary thyroid carcinoma is primary. Furthermore, the kit in accordance with the present invention can serve as a kit for providing a criterion for determining whether or not a tumor metastasized to cervical lymph node is of thyroid origin.

As has been described, an object of the present invention is to provide a tumor marker, and to provide a diagnostic criterion for papillary thyroid carcinoma by the detection of the tumor marker. A person skilled in the art who has read this Description easily understands on the basis of the Description and common technical knowledge that it is possible to implement the present invention in various modes.

### Examples

### [1. Obtaining human SNX5 cDNA]

RT-PCR was carried out using, as a template, a total RNA extracted from a human HacaT cell, and using a forward primer (pCMV-HA-SNX5 FW: 5'-CAGGCCCGAATTCGGATGGCCGCGGTTCCCGAG-3' (SEQ ID NO:4)) and a reverse primer (pCMV-HA-SNX5 RV: 5'-GATCTCGGTCGACCGTGAAGGCATATCAGTTAT-3' (SEQ ID NO:5)), thereby obtaining human SNX5 cDNAs. The human SNX5 cDNAs thus obtained were inserted into an expression vector pET3c (Novagen) for *Escherichia coli* and an expression vector pCMV-HA (BD Bioscience) for mammals to obtain pET3c-SNX5 and pCMV-HA-SNX5, respectively. Note that pCMV-HA-SNX5 FW is obtained by linkage of a restriction site etc. to a sequence (ATGGCCGCGGTTCCCGAG (SEQ ID NO:6)) in the 5' region of the human SNX5 cDNA, and pCMV-HA-SNX5 RV is obtained by linkage of a restriction site etc. to a sequence complementary to a sequence (ataactgatatgccttcac (SEQ ID NO:7)) in the 3' region of the human SNX5 cDNA.

### [2. Production of anti-human SNX5 monoclonal antibody]

An *Escherichia coli* BL21 to which pET3c-SNX5 has been introduced was cultivated, and protein synthesis was induced by IPTG. Then, a human SNX5 protein was separated from bacterial cell components, and concentrated. Using an obtained protein as immunogen, 6 to 8 week-old Balb/c mice were immunized intraperitoneally (100 µg for each mouse) by using, as adjuvant, a complete adjuvant (for first immunization only) or an incomplete adjuvant (for second and later immunizations). Further immunization was carried out every other week for two months, and final immunization was carried out three days before the day on which splenocyte was collected. The splenocyte thus collected was fused to a NSO mouse myeloma cell with use of polyethyleneglycol, and a fused cell thus obtained was cultivated in a 96 well plate with a PRM11640 culture medium containing HAT and 10% FBS for two to three weeks. Western blotting was carried out using a supernatant to screen for an anti-human SNX5 monoclonal antibody.

### [3. Gene transfer into mammalian cell]

To a human 293 cell cultivated in DMEM containing 10% FBS and penicillin/streptomycin, pCMV-SNX5 was introduced with use of LF2000 (manufactured by Invitrogen) in accordance with the manufacturer's guidebook. Note here that expression of SNX5 in a transformant was confirmed by RT-PCR using, as a template, a total RNA extracted from the transformant, and using a forward primer (SNX5 AMP FW: 5'-ccggttaaagagcaaagacg-3' (SEQ ID NO:8)) and a reverse primer (SNX5 AMP RV: 5'-agctctgcaaaagggagaca-3' (SEQ ID NO:9)).

### [4. Western blot analysis]

A human 293 cell into which pCMV-SNX5 has been introduced was cultivated for three days, and thereafter, was solubilized with a buffer solution containing 0.5% NP-40. A protein in a solubilized fraction was separated by SDS-PAGE using 5% to 20% gradient gel. The protein thus separated was transferred to a nylon membrane, and a primary antibody reaction using an anti-human SNX5 monoclonal antibody and a secondary antibody reaction using an peroxidase-conjugated goat anti-mouse IgG antibody were each carried out for 1 hour. Signals were visualized by using an ECL kit (manufactured by Amersham plc).

See Fig. 1 for the results. Although nonspecific bands were detected at 50 kDa, a signal specific to SNX5 (molecular weight: 47 kDa) was detected only in the cell into which pCMV-SNX5 has been introduced. This signal was not detected in the cells into which negative controls (CMV, pCMV-mutant AIRE#1, and pCMV-mutant AIRE#2) have been introduced.

### [5. Immunohistochemical staining]

A human 293 cell into which pCMV-SNX5 has been introduced was cultivated for three days, and thereafter, fixed with paraformaldehyde. Then, immunohistochemical staining was carried out by reacting the human 293 cells (i) for 1 hour at room temperature by using an anti-human SNX5 monoclonal antibody as a primary antibody and (ii) for 1 hour at room temperature by using, as a secondary antibody, a goat anti-mouse IgG antibody complexed with Alexa 596. Signals were detected by using IX71 fluorescence microscope (manufactured by OLYMPUS CORPORATION). Fig. 2 shows the cells to which pCMV-SNX5 has been introduced (left) and the cells to which pCMV has been introduced, which cells serve as negative controls (right). Fig. 2 shows that the anti-human SNX5 monoclonal antibody reacts specifically with SNX5.

Further, immunohistochemical staining was carried out with respect to formalin-fixed, paraffin-embedded sections of adenocarcinoma tissues by using an anti-human SNX5 monoclonal antibody. Signals were detected (see Fig. 3) by using an automated immunostaining device (manufactured by DAKO). As shown in (a) of Fig. 3, strong expression of SNX5 was detected in papillary thyroid carcinoma. However, no expression of SNX5 was detected ((b) of Fig. 3) in lung adenocarcinoma ((b) of Fig. 3) and in mammary adenocarcinoma ((c) of Fig. 3).

Furthermore, the distribution of SNX5 expression in tumor tissue, which distribution was studied by using an established anti-human SNX5 monoclonal antibody, was verified by using a commercially available rabbit anti-human SNX5 polyclonal antibody (H40: Santa Cruz Biotechnology) (Fig. 4). As shown in Fig. 4, strong expression of SNX5 was detected in tumor cells when immunohistochemical staining was carried out with respect to a formalin-fixed, paraffin-embedded section of papillary thyroid carcinoma.

Moreover, SNX5 expression in various malignant tumors that show papillary proliferation was analyzed by using a mouse anti-human SNX5 monoclonal antibody. The results are shown in Table 1. As shown in Table 1, no expression of SNX5 was detected in any of 20 cases of malignant epithelial tumors (including gastric carcinoma, colon carcinoma and pancreatic carcinoma) other than those in a thyroid gland and 5 cases of sarcoma and malignant nonepithelial tumors.

**Table 1**

| Tumor tissue | SNX5-positive case | SNX5-negative case | Total |
|---|---|---|---|
| Papillary thyroid carcinoma (Primary) | 19 | 1 | 20 |
| Papillary thyroid carcinoma (Metastasis in lymph node) | 11 | 1 | 12 |
| Lung papillary adenocarcinoma | 0 | 5 | 5 |
| Papillotubular carcinoma in mammary gland | 0 | 4 | 4 |
| Malignant epithelial tumors other than those in thyroid gland | 0 | 20 | 20 |
| Sarcoma and malignant nonepithelial tumors | 0 | 5 | 5 |

### [6. Quantitative PCR]

A total RNA was extracted from a normal thyroid tissue collected from a subject via biopsy or was extracted from a thyroid carcinoma tissue extirpated by surgery from a patient with thyroid carcinoma, and cDNA was produced by using a reverse transcriptase (Invitrogen). Then, quantitative PCR (Applied Biosystems) was carried out using this cDNA as a template and using a PCR probe (product number: Hs00429583). Obtained values were analyzed by using ribosomal RNA as a control, and were subjected to comparative study by a delta delta CT method (AMI 7000, Applied Biosystems). It should be noted that genetic analysis of human tissues was carried out only for analysis of expression of SNX5, and the studies using human tissues were carried out with the approval of the ethical committee after sufficient informed consent was obtained. The studies were carried out while extreme care was taken to protect data and to protect human rights, in compliance with laws.

Fig. 5 illustrates the results for 3 cases of thyroid carcinoma. As illustrated in Fig. 5, it was confirmed that the expression of SNX5 gene is higher in thyroid carcinoma tissues than in normal thyroid carcinoma tissues.

As has been described, the inventors of the present invention have for the first time found that SNX5 is highly expressed in papillary thyroid carcinoma. Further, the mouse anti-human SNX5 monoclonal antibody established by the inventors of the present invention allows for immunohistologic analyses of not only formalin-fixed, paraffin-embedded sections, but also paraform-fixed tissues. According to the information obtained through the present t invention, SNX5 is considered as an excellent marker as compared to known thyroid markers such as thyroglobulin or TTF-1, and is considered as being capable of providing useful information when pathological diagnosis is made. Further, it is considered that it is possible to make serodiagnosis of a neoplastic lesion of thyroid origin, by for example ELISA using an anti-human SNX5 monoclonal antibody.

The present invention is not limited to the descriptions of the respective embodiments, but may be altered within the scope of the claims. An embodiment derived from a proper combination of technical means disclosed in different embodiments is encompassed in the technical scope of the invention.

Further, all the academic literatures and patent literatures listed in this Description are incorporated in this Description as references.

### Industrial Applicability

By use of the present invention, it is possible to easily carry out early and definitive diagnoses of papillary thyroid carcinoma. The present invention providing such an excellent tool is usable in the fields of medicine and pharmacy, and is capable of significantly contributing to the development of pharmaceuticals and biochemical regents.

## Claims

1. A method of detecting a tumor marker for papillary thyroid carcinoma, comprising the step of detecting a SNX5 protein or a fragment thereof in a sample from a subject.

2. The method according to claim 1, wherein the step of detecting is carried out by an immunoassay using an anti-SNX5 antibody.

3. The method according to claim 2, wherein the anti-SNX5 antibody is a mouse anti-human SNX5 monoclonal antibody produced by a hybridoma 48C2 or a monoclonal antibody having a binding activity equivalent to that of the mouse anti-human SNX5 monoclonal antibody.

4. A method of detecting a tumor marker for papillary thyroid carcinoma, comprising the step of detecting a SNX5 gene or a fragment thereof in a sample from a subject.

5. The method according to claim 4, wherein the step of detecting is carried out by hybridizing a nucleic acid probe with the SNX5 gene or the fragment thereof.

6. The method according to claim 5, wherein the nucleic acid probe is an oligonucleotide having a nucleotide sequence shown in any one of SEQ ID NOs:4-9 or having a sequence complementary to the nucleotide sequence.

7. The method according to any one of claims 1 through 6, wherein the sample is a section or a cell lysate, which is prepared from a tissue collected from a subject or a cell of the tissue.

8. The method according to claim 7, wherein the tissue is a thyroid tissue.

9. The method according to any one of claims 1 through 8, wherein the subject is a patient with suspected thyroid disease.

10. A kit for detecting a tumor marker for papillary thyroid carcinoma, comprising an anti-SNX5 antibody.

11. The kit according to claim 10, wherein the anti-SNX5 antibody is a mouse anti-human SNX5 monoclonal antibody produced by a hybridoma 48C2 or a monoclonal antibody having a binding activity equivalent to that of the mouse anti-human SNX5 monoclonal antibody.

12. A kit for detecting a tumor marker for papillary thyroid carcinoma, comprising an oligonucleotide that can hybridize with a SNX5 gene or a fragment thereof.

13. The kit according to claim 12, wherein the oligonucleotide has a nucleotide sequence shown in any one of SEQ ID NOs:4-9 or has a sequence complementary to the nucleotide sequence.


SEQUENCE LISTING

<110> Sapporo Medical University

<120> A tumor marker and use thereof

<130> PM1015

<150> JP 2009-095561
<151> 2009-04-10

<160> 10

<170> PatentIn Ver. 2.1

<210> 1
<211> 404
<212> PRT
<213> human

<400> 1
Met Ala Ala Val Pro Glu Leu Leu Gln Gln Gln Glu Glu Asp Arg Ser
  1               5                  10                  15

Lys Leu Arg Ser Val Ser Val Asp Leu Asn Val Asp Pro Ser Leu Gln
             20                  25                  30

Ile Asp Ile Pro Asp Ala Leu Ser Glu Arg Asp Lys Val Lys Phe Thr
         35                  40                  45

Val His Thr Lys Thr Thr Leu Pro Thr Phe Gln Ser Pro Glu Phe Ser
     50                  55                  60

Val Thr Arg Gln His Glu Asp Phe Val Trp Leu His Asp Thr Leu Ile
 65                  70                  75                  80

Glu Thr Thr Asp Tyr Ala Gly Leu Ile Ile Pro Pro Ala Pro Thr Lys
                 85                  90                  95

Pro Asp Phe Asp Gly Pro Arg Glu Lys Met Gln Lys Leu Gly Glu Gly
            100                 105                 110

Glu Gly Ser Met Thr Lys Glu Glu Phe Ala Lys Met Lys Gln Glu Leu
        115                 120                 125

Glu Ala Glu Tyr Leu Ala Val Phe Lys Lys Thr Val Ser Ser His Glu
    130                 135                 140

Val Phe Leu Gln Arg Leu Ser Ser His Pro Val Leu Ser Lys Asp Arg
145                 150                 155                 160

Asn Phe His Val Phe Leu Glu Tyr Asp Gln Asp Leu Ser Val Arg Arg
                165                 170                 175

Lys Asn Thr Lys Glu Met Phe Gly Gly Phe Phe Lys Ser Val Val Lys
            180                 185                 190

Ser Ala Asp Glu Val Leu Phe Thr Gly Val Lys Glu Val Asp Asp Phe
        195                 200                 205

Phe Glu Gln Glu Lys Asn Phe Leu Ile Asn Tyr Tyr Asn Arg Ile Lys
    210                 215                 220

Asp Ser Cys Val Lys Ala Asp Lys Met Thr Arg Ser His Lys Asn Val

225                 230                 235                 240

Ala Asp Asp Tyr Ile His Thr Ala Ala Cys Leu His Ser Leu Ala Leu
                245                 250                 255

Glu Glu Pro Thr Val Ile Lys Lys Tyr Leu Leu Lys Val Ala Glu Leu
            260                 265                 270

Phe Glu Lys Leu Arg Lys Val Glu Gly Arg Val Ser Ser Asp Glu Asp
        275                 280                 285

Leu Lys Leu Thr Glu Leu Leu Arg Tyr Tyr Met Leu Asn Ile Glu Ala
    290                 295                 300

Ala Lys Asp Leu Leu Tyr Arg Arg Thr Lys Ala Leu Ile Asp Tyr Glu
305                 310                 315                 320

Asn Ser Asn Lys Ala Leu Asp Lys Ala Arg Leu Lys Ser Lys Asp Val
                325                 330                 335

Lys Leu Ala Glu Ala His Gln Gln Glu Cys Cys Gln Lys Phe Glu Gln
            340                 345                 350

Leu Ser Glu Ser Ala Lys Glu Glu Leu Ile Asn Phe Lys Arg Lys Arg
        355                 360                 365

Val Ala Ala Phe Arg Lys Asn Leu Ile Glu Met Ser Glu Leu Glu Ile
    370                 375                 380

Lys His Ala Arg Asn Asn Val Ser Leu Leu Gln Ser Cys Ile Asp Leu
385                 390                 395                 400

Phe Lys Asn Asn



<210> 2
<211> 1215
<212> DNA
<213> human

<400> 2
atggccgcgg ttcccgagtt gctgcagcag caggaggagg accgcagcaa gctgagatct 60
gtatctgtgg acctgaatgt tgatccctcg cttcagattg acatacctga tgcgctcagt 120
gagagagaca aagtcaaatt tacagtgcac acaaagacca cactgcccac gtttcagagc 180
ccagagtttt ctgttacaag gcaacatgaa gactttgtgt ggctacatga cactcttatt 240
gaaacaacag actatgctgg gcttattatt ccacctgctc ctacgaagcc cgactttgat 300
ggtcctcgag agaagatgca gaaactggga gaaggtgaag ggtctatgac caaagaagaa 360
tttgccaaga tgaaacaaga actggaagct gagtatctcg ctgtgtttaa gaagactgtg 420
tcctcccatg aagtctttct tcagcggctt tcttctcacc ctgttctcag taaagatcgc 480
aactttcatg ttttcctgga atatgatcag gatctaagtg ttaggcggaa aaatactaaa 540
gagatgtttg gtggcttctt caaaagtgtg gtgaaaagtg ctgatgaagt cctttttact 600
ggagttaagg aggtagatga cttctttgag caagagaaga acttccttat taactattac 660
aataggatca aagattcttg tgtgaaagct gacaaaatga ccagatctca taaaaatgtt 720
gccgatgact atatccacac cgcagcctgc ttacatagcc tggctttaga agagcccaca 780
gtcatcaaaa agtacctatt gaaggttgct gagctatttg aaaaactaag gaaagtagag 840
ggtcgagttt catcagatga agatttgaag ctaacagagc tcctccgata ctacatgctc 900
aacattgaag ctgctaagga tctcttatac agacgcacca aagccctcat tgactatgag 960
aactcaaaca aagctctgga taaggcccgg ttaaagagca aagacgtcaa gttggctgag 1020
gcacaccagc aggagtgctg ccagaaattt gaacaacttt ccgaatctgc aaaagaagaa 1080
ctgataaatt tcaaacggaa gagagtggca gcatttagaa agaatctaat tgaaatgtct 1140
gaactggaaa taaaacatgc caggaacaat gtctcccttt tgcagagctg tattgacttg 1200
ttcaagaata actga                                                  1215

<210> 3
<211> 1547
<212> DNA
<213> human

<400> 3
tgccatcttg gactcgggct gagtaataaa ttccccccgc accgaaacgc ggtctttctc 60
tagacgcgtc ttgctgggag agtgtccgtt gcttcccgtc cgtgtcgcgg ccctgcggtt 120
ggcggcctcc tcgtggagcg gagcaaggcc aggcggcccc tgctcgagtc ccgcgtcgcc 180
atggccgcgg ttcccgagtt gctgcagcag caggaggagg accgcagcaa gctgagatct 240
gtatctgtgg acctgaatgt tgatccctcg cttcagattg acatacctga tgcgctcagt 300
gagagagaca aagtcaaatt tacagtgcac acaaagacca cactgcccac gtttcagagc 360
ccagagtttt ctgttacaag gcaacatgaa gactttgtgt ggctacatga cactcttatt 420
gaaacaacag actatgctgg gcttattatt ccacctgctc ctacgaagcc cgactttgat 480
ggtcctcgag agaagatgca gaaactggga gaaggtgaag ggtctatgac caaagaagaa 540
tttgccaaga tgaaacaaga actggaagct gagtatctcg ctgtgtttaa gaagactgtg 600
tcctcccatg aagtctttct tcagcggctt tcttctcacc ctgttctcag taaagatcgc 660
aactttcatg ttttcctgga atatgatcag gatctaagtg ttaggcggaa aaatactaaa 720
gagatgtttg gtggcttctt caaaagtgtg gtgaaaagtg ctgatgaagt cctttttact 780
ggagttaagg aggtagatga cttctttgag caagagaaga acttccttat taactattac 840
aataggatca aagattcttg tgtgaaagct gacaaaatga ccagatctca taaaaatgtt 900
gccgatgact atatccacac cgcagcctgc ttacatagcc tggctttaga agagcccaca 960
gtcatcaaaa agtacctatt gaaggttgct gagctatttg aaaaactaag gaaagtagag 1020
ggtcgagttt catcagatga agatttgaag ctaacagagc tcctccgata ctacatgctc 1080
aacattgaag ctgctaagga tctcttatac agacgcacca aagccctcat tgactatgag 1140
aactcaaaca aagctctgga taaggcccgg ttaaagagca aagacgtcaa gttggctgag 1200
gcacaccagc aggagtgctg ccagaaattt gaacaacttt ccgaatctgc aaaagaagaa 1260
ctgataaatt tcaaacggaa gagagtggca gcatttagaa agaatctaat tgaaatgtct 1320
gaactggaaa taaaacatgc caggaacaat gtctcccttt tgcagagctg tattgacttg 1380
ttcaagaata actgatatgc cttcactcag aagaaaagaa atgaatgtga aagaaagcca 1440
agcatcactt gcacttaaat cattaccacg gaagatatat tagcttcaac tttagtttaa 1500
aattatgtga ataaatattt tgatttctac aaatcttaac atttaaa 1547

<210> 4
<211> 33
<212> DNA
<213> Artificial Sequence

<220>
<223> Description of Artificial Sequence: primer

<400> 4
caggcccgaa ttcggatggc cgcggttccc gag 33

<210> 5
<211> 33
<212> DNA
<213> Artificial Sequence

<220>
<223> Description of Artificial Sequence: primer

<400> 5
gatctcggtc gaccgtgaag gcatatcagt tat 33

<210> 6
<211> 18
<212> DNA
<213> Artificial Sequence

<220>
<223> Description of Artificial Sequence: primer

<400> 6
atggccgcgg ttcccgag                                                    18


<210> 7
<211> 19
<212> DNA
<213> Artificial Sequence

<220>
<223> Description of Artificial Sequence: primer

<400> 7
ataactgata tgccttcac                                                   19


<210> 8
<211> 20
<212> DNA
<213> Artificial Sequence

<220>
<223> Description of Artificial Sequence: primer

<400> 8
ccggttaaag agcaaagacg                                                  20


<210> 9
<211> 20
<212> DNA
<213> Artificial Sequence

<220>
<223> Description of Artificial Sequence: primer

<400> 9
agctctgcaa aagggagaca                                                  20


<210> 10
<211> 177
<212> PRT
<213> human

<400> 10
Met Ala Ala Val Pro Glu Leu Leu Gln Gln Gln Glu Glu Asp Arg Ser
  1               5                  10                  15

Lys Leu Arg Ser Val Ser Val Asp Leu Asn Val Asp Pro Ser Leu Gln
             20                  25                  30

Ile Asp Ile Pro Asp Ala Leu Ser Glu Arg Asp Lys Val Lys Phe Thr
         35                  40                  45

Val His Thr Lys Thr Thr Leu Pro Thr Phe Gln Ser Pro Glu Phe Ser
     50                  55                  60

Val Thr Arg Gln His Glu Asp Phe Val Trp Leu His Asp Thr Leu Ile
 65                  70                  75                  80

Glu Thr Thr Asp Tyr Ala Gly Leu Ile Ile Pro Pro Ala Pro Thr Lys
                 85                  90                  95

Pro Asp Phe Asp Gly Pro Arg Glu Lys Met Gln Lys Leu Gly Glu Gly
            100                 105                 110

Glu Gly Ser Met Thr Lys Glu Glu Phe Ala Lys Met Lys Gln Glu Leu
        115                 120                 125

Glu Ala Glu Tyr Leu Ala Val Phe Lys Lys Thr Val Ser Ser His Glu
    130                 135                 140

Val Phe Leu Gln Arg Leu Ser Ser His Pro Val Leu Ser Lys Asp Arg
145                 150                 155                 160

Asn Phe His Val Phe Leu Glu Tyr Asp Gln Asp Leu Ser Val Arg Arg
                165                 170                 175

Lys